(19) Europäisches Patentamt
European Patent Office
Office européen des brevets

(11) Veröffentlichungsnummer: **0 120 404**
**B1**

(12) # EUROPÄISCHE PATENTSCHRIFT

(45) Veröffentlichungstag der Patentschrift: **19.02.86**

(51) Int. Cl.⁴: **C 07 C 17/00**, C 07 C 21/24, C 07 C 25/02, C 07 C 79/12, C 07 C 15/52

(21) Anmeldenummer: **84102748.5**

(22) Anmeldetag: **14.03.84**

(54) **Verfahren zur Herstellung von Benzalchloriden.**

(30) Priorität: **25.03.83 DE 3310953**

(43) Veröffentlichungstag der Anmeldung: **03.10.84 Patentblatt 84/40**

(45) Bekanntmachung des Hinweises auf die Patenterteilung: **19.02.86 Patentblatt 86/8**

(84) Benannte Vertragsstaaten: **BE CH DE FR GB LI**

(56) Entgegenhaltungen:
**DE - B - 2 139 779**
**FR - A - 1 551 559**

**IZVESTIYA AKADEMI NAUK SSSR, SERIA KHIMICHESKAYA, Nr. 7, 1970 (englische Übersetzung) R.G. PETROVA und R. KH. FREIDLINA "The reduction of alpha, alpha, alpha, w-tetrachloroalkanes by the action of thiols in the presence of iron compounds" Seiten 1438-1485**
**TETRAHEDRON LETTERS, Nr. 48, 1968, London I.M. DOWNIE und J.B. LEE "Reduction of perhalocompounds with tertiary phosphines and phosphorous tris(di-n-alkyl) amides" Seiten 4951, 4952**
**PATENTS ABSTRACTS OF JAPAN, unexamined applications, Sektion C, Band 2, Nr. 140, 18. November**

(73) Patentinhaber: **BAYER AG, Konzernverwaltung RP Patentabteilung, D-5090 Leverkusen 1 Bayerwerk (DE)**

(72) Erfinder: **Baasner, Bernd, Dr., Mozartstrasse 41, D-5090 Leverkusen 1 (DE)**
Erfinder: **Klauke, Erich, Dr., Eichendorffweg 8, D-5068 Odenthal (DE)**

(56) Entgegenhaltungen: (Fortsetzung)
**1978 THE PATENT OFFICE JAPANESE GOVERNMENT Seite 3092 C 78**
**CESARE FERRI "Reaktionen in der organischen Synthese", 1978 GEORG THIEME VERLAG, Stuttgart, Seite 111**

**EP 0 120 404 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Benzalchloriden aus Benzotrichloriden.

Benzalchloride sind wichtige Zwischenprodukte, die beispielsweise durch Verseifung in die entsprechenden Benzaldehyde überführt werden können. Benzaldehyde werden beispielsweise zur Herstellung von Herbiziden verwendet (s. US-P 4212998 und US-P 4212999).

Es ist bereits bekannt, dass man Benzotrichloride in Benzalchloride überführen kann. Beispielsweise kann man Benzotrichlorid mit trivalenten Phosphorverbindungen zu Benzalchlorid umsetzen. Gute Ergebnisse werden dabei nur in speziellen Fällen erhalten, beispielsweise wenn man als trivalente Phosphorverbindung Phosphorigsäure-tris-(di-N-ethyl)-amid in Ether einsetzt und in Gegenwart von Ethanol arbeitet (s. I.M. Downie und J.B. Lee, Tetrahedron letters *1968*, 4951).

An Aliphaten gebundene $CCl_3$-Gruppen kann man mit Nickeltetracarbonyl in Tetrahydrofuran zu $CHCl_2$-Gruppen umsetzen. Dieses Verfahren ist jedoch nicht auf an Aromaten gebundene $CCl_3$-Gruppen zu übertragen, da dann keine Benzalchloride gebildet werden, sondern eine dehalogenierende Dimerisierung unter Bildung von Diphenylethanderivaten eintritt (s. T. Kunieda et al., J.C.S. Chem. Comm. *1972*, 885).

Ein weiteres Verfahren zur Überführung von an Aliphaten gebundene $CCl_3$-Gruppen in $CHCl_2$-Gruppen wendet Thiole als Reduktionsmittel an und wird in Gegenwart von Eisencarbonylen oder Eisenchloriden durchgeführt [s. R.G. Petrova und R.Kh. Freidlina, Izvest. Akad. Nauk. SSSR *1970*, 1483 (engl.)]. Über die Übertragbarkeit dieses Verfahrens auf an Aromaten gebundene $CCl_3$-Gruppen ist nichts bekannt geworden. Es muss auch hier damit gerechnet werden, dass sich im Falle des Einsatzes von an Aromaten gebundenen $CCl_3$-Gruppen Diphenylethanderivate bilden und/oder Friedel-Crafts-Reaktionen ablaufen.

Die Umsetzung von Trichloressigsäure zu Dichloressigsäure mit metallischem Kupfer als Reduktionsmittel ist aus Cesare Ferri, „Reaktionen der organischen Synthese", Thieme-Verlag, Stuttgart 1978, S. 111, bekannt.

Es wurde nun ein Verfahren zur Herstellung von Benzalchloriden aus Benzotrichloriden gefunden, das dadurch gekennzeichnet ist, dass man Benzotrichloride mit Thiolen in Gegenwart von Kupfer-(I)-bromid bei erhöhten Temperaturen umsetzt.

Zum Einsatz in das erfindungsgemässe Verfahren sind die verschiedensten Benzotrichloride geeignet. Es kann sich dabei um unsubstituiertes Benzotrichlorid ($C_6H_5CCl_3$) oder um am aromatischen Kern einfach oder mehrfach substituierte Benzotrichloride handeln.

Geeignete Benzotrichloride sind beispielsweise solche, die der Formel

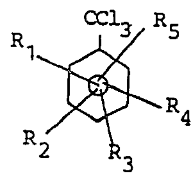

(I)

entsprechen, in der

$R_1$ bis $R_5$ unabhängig voneinander für Wasserstoff, Nitro, Halogen, Alkyl, Fluoralkyl, Aryl, substituiertes Aryl, O-Alkyl, O-Fluoralkyl, O-Aryl, O-substituiertes Aryl und/oder Cyanid stehen.

Halogenreste sind dabei vorzugsweise Fluor und Chlor. Alkyl-, Fluoralkyl-, O-Alkyl- und O-Fluoralkyl-Reste enthalten dabei vorzugsweise 1 bis 12 C-Atome, besonders bevorzugt 1 bis 4 C-Atome. Aryl-, substituierte Aryl-, O-Aryl- und O-substituiertes Aryl-Reste enthalten dabei vorzugsweise 6 bis 10 C-Atome, besonders bevorzugt 6 bis 8 C-Atome.

Bevorzugt werden in das erfindungsgemässe Verfahren unsubstituiertes Benzotrichlorid oder am aromatischen Kern 1-, 2- oder 3fach substituierte Benzotrichloride eingesetzt. Bevorzugte Benzotrichloride sind beispielsweise solche, die der Formel

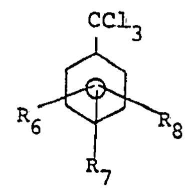

(II)

entsprechen, in der

$R_6$ bis $R_8$ unabhängig voneinander für Wasserstoff, Nitro, Fluor, Chlor, Cyanid, $C_1$-$C_4$-Alkyl, $C_1$-$C_4$-Fluoralkyl und/oder O-$C_1$-$C_4$-Fluoralkyl stehen.

Besonders bevorzugt werden in das erfindungsgemässe Verfahren Benzotrichloride der Formel (II) eingesetzt, in der $R_6$ bis $R_8$ unabhängig voneinander für Wasserstoff, Nitro, Fluor, Chlor, Cyanid, Methyl, Trifluormethyl und/oder o-Trifluormethyl stehen.

Ganz besonders bevorzugt werden in das erfindungsgemässe Verfahren Benzotrichlorid, o-Chlorbenzotrichlorid, p-Chlorbenzotrichlorid, o,p-Dichlorbenzotrichlorid, m-Fluorbenzotrichlorid, p-Fluorbenzotrichlorid, o-Fluor-o'-chlorbenzotrichlorid, o,o'-Difluorbenzotrichlorid, m-Methylbenzotrichlorid, o-Trifluormethylbenzotrichlorid, m-Trifluormethylbenzotrichlorid, p-Trifluormethylbenzotrichlorid, m-Oxitrifluormethylbenzotrichlorid, o-Chlor-m-trifluormethylbenzotrichlorid, m-Nitro-m'-trifluormethyl-p-chlorbenzotrichlorid, o-Cyano-benzotrichlorid, m-Cyano-benzotrichlorid und p-Cyano-benzotrichlorid eingesetzt.

Zum Einsatz in das erfindungsgemässe Verfahren sind die verschiedensten Thiole geeignet. Vorzugsweise werden Thiole eingesetzt, die unter den Reaktionsbedingungen flüssig sind. Andere Thiole müssen in gelöster Form eingesetzt werden. Bevorzugt werden aliphatische Thiole mit 4 oder mehr C-Atomen und aromatische Thiole mit 6 oder mehr C-Atomen eingesetzt, beispielsweise aliphatische Thiole mit 4 bis 10 C-Atomen oder aromatische Thiole mit 6 bis 8 C-Atomen. Besonders bevorzugt ist der Einsatz von Butanthiol und Thiophenol. Der Einsatz von Thiophenol ist ganz besonders bevorzugt.

Für die Umsetzung von 1 Mol eines Benzotrichlorids zu dem entsprechenden Benzalchlorid werden 2 Mol Thiol benötigt. Man setzt deshalb in das erfindungsgemässe Verfahren vorzugsweise pro Mol eines Benzotrichlorids 2 Mol eines Thiols ein, da dann praktisch kein unumgesetztes Benzotrichlorid oder unumgesetztes Thiol aus dem Reaktionsgemisch abgetrennt werden muss. Das erfindungsgemässe Verfahren kann aber auch bei kleineren oder grösseren Molverhältnissen von Benzotrichloriden zu Thiolen als 1:2 durchgeführt werden.

Das erfindungsgemässe Verfahren wird in Gegenwart von Kupfer-(I)-bromid durchgeführt.

Es ist ausreichend, wenn das Kupfer-(I)-bromid in geringen Mengen zugegen ist. Geeignete Mengen sind beispielsweise solche von 0,01 bis 10 Gew.-%, bevorzugt sind solche von 0,1 bis 5 Gew.-%, besonders bevorzugt solche von 0,5 bis 2 Gew.-%, jeweils bezogen auf das eingesetzte Benzotrichlorid.

Das erfindungsgemässe Verfahren wird im allgemeinen in Abwesenheit von Lösungsmitteln durchgeführt. Es kann aber auch in Gegenwart von Lösungsmitteln durchgeführt werden. Die Gegenwart von Lösungsmitteln ist beispielsweise dann erforderlich, wenn Thiole eingesetzt werden sollen, die unter den Reaktionsbedingungen nicht flüssig sind. Geeignete Lösungsmittel sind beispielsweise solche, die einen Schmelzpunkt von weniger als 80°C und einen Siedepunkt von über 100°C aufweisen, hinreichend inert sind und ein ausreichendes Lösungsvermögen für das jeweils eingesetzte Benzotrichlorid und/oder Thiol aufweisen. Im allgemeinen sind Toluol, Xylol, Mesitylen und Methylcyclohexan gut geeignete Lösungsmittel.

Während der erfindungsgemässen Umsetzung spaltet sich Chlorwasserstoff ab. Die Temperaturuntergrenze für das erfindungsgemässe Verfahren ist deshalb die Temperatur, bei der im jeweiligen Fall die Chlorwasserstoffentwicklung einsetzt. Im allgemeinen liegt diese Temperatur bei etwa 80°C, sie kann in speziellen Fällen auch höher oder tiefer liegen, beispielsweise im Bereich von 50 bis 120°C. Die Temperaturobergrenze für das erfindungsgemässe Verfahren ist weniger kritisch. Aus praktischen Erwägungen arbeitet man im allgemeinen höchstens bei der Temperatur, bei der das Reaktionsgemisch unter Rückfluss siedet, wobei man diese Temperatur, z.B. durch entsprechende Wahl des eingesetzten Thiols, eines Lösungsmittels und/oder des Druckes im Reaktionsgefäss beeinflussen kann. Bevorzugt führt man das erfindungsgemässe Verfahren bei Temperaturen im Bereich von 50 bis 150°C, besonders bevorzugt bei Temperaturen im Bereich von 80 bis 120°C, durch. Beispielsweise kann man hierbei so verfahren, dass man das Reaktionsgemisch zunächst auf 80°C erhitzt und dann die Temperatur langsam auf 120°C steigert und so lange auf 120°C hält, bis die Chlorwasserstoff-Entwicklung beendet ist.

Das erfindungsgemässe Verfahren wird im allgemeinen bei Normaldruck durchgeführt. Es kann auch bei erhöhtem oder erniedrigtem Druck durchgeführt werden, beispielsweise im Druckbereich von 0,5 bis 20 bar. Ein Arbeiten bei einem erniedrigten Druck kann vorteilhaft sein, wenn man die Chlorwasserstoffabspaltung und -entfernung erleichtern will. Ein Arbeiten bei einem erhöhten Druck kann vorteilhaft sein, wenn man die Reaktion bei Temperaturen durchführen, möchte die höher liegen als der Siedepunkt der bei Normaldruck am niedrigsten siedenden Komponente des Reaktionsgemisches.

Das Reaktionsgemisch wird im allgemeinen so lange auf Reaktionstemperatur gehalten, bis die Chlorwasserstoffentwicklung beendet ist. Diese Zeit kann beispielsweise im Bereich von 2 bis 15 h liegen, meist liegt sie im Bereich zwischen 5 und 10 h.

Nach Beendigung der Reaktion kann das Reaktionsgemisch beispielsweise durch Destillation aufgearbeitet werden.

Man kann hier so verfahren, dass man, sofern ein Lösungsmittel vorhanden ist, nach dessen Abtrennung das erhaltene Benzalchlorid durch Destillation bei vermindertem Druck aus dem Reaktionsgemisch entfernt. Geeignete Drücke sind dabei beispielsweise solche im Bereich von 0,1 bis 50 mbar. In der Regel fallen dabei die Benzalchloride bereits in einer für weitere Umsetzungen ausreichenden Reinheit an. Die so abgetrennten Benzalchloride können gegebenenfalls zur weiteren Reinigung nochmals destilliert werden. Dies ist insbesondere dann von Vorteil, wenn die zunächst abgetrennten Benzalchloride durch störende Mengen an unumgesetzten Benzotrichloriden und/oder aus den Thiolen entstandenen Disulfiden verunreinigt sind, was dann der Fall sein kann, wenn die Siedepunkte von eingesetztem Benzotrichlorid, gebildetem Benzalchlorid und/oder gebildetem Disulfid nahe beieinanderliegen.

Die im allgemeinen im Destillationsrückstand verbleibenden Disulfide können auf bekannte Weise durch katalytische Hydrierung in die entsprechenden Thiole rücküberführt werden, die dann erneut in die erfindungsgemässe Umsetzung eingesetzt werden können.

Das erfindungsgemässe Verfahren gestattet die Herstellung der verschiedensten Benzalchloride aus den entsprechenden Benzotrichloriden in einfacher Weise und mit guten Ausbeuten. Es ist ausgesprochen überraschend, dass dabei die Bildung von Diphenylethanderivaten, wenn überhaupt, dann nur in sehr untergeordnetem Masse eintritt. Die Bildung von Diphenylethanderivaten erfolgt allenfalls in Spuren und liegt maximal bei 2%. Die Vorteile des erfindungsgemässen Verfahrens sind am ausgeprägtesten, wenn man als Thiol Thiophenol einsetzt.

Die folgenden Beispiele erläutern das erfindungsgemässe Verfahren, ohne es zu beschränken.

*Beispiele 1 bis 18*

1 Mol des jeweils angegebenen Benzotrichlorides, 2 Mol Thiophenol und 2 g Kupfer-(I)-bromid wurden unter Rühren erhitzt. Bei etwa 80°C begann die Entwicklung von Chlorwasserstoff und die Reaktion verlief dann leicht exotherm. Die Temperatur wurde langsam auf 120°C erhöht und die Reaktion bei 120°C zu Ende geführt. Danach wurde das jeweils erhaltene Benzalchlorid bei vermindertem Druck aus dem Reaktionsgemisch abdestilliert. Weitere Einzelheiten sind in der folgenden Tabelle angegeben.

| Beispiel Nr. | Einsatzprodukt (BTC=Benzotrichlorid) | Reaktionsprodukt (BAC=Benzalchlorid) | Reaktionszeit (h) | Siedepunkt (°C) (Druck mbar) bei Destillation | Ausbeute (% der Theorie) |
|---|---|---|---|---|---|
| 1 | BTC | BAC | 8 | 104-105/30 | 82 |
| 2 | o-Chlor-BTC | o-Chlor-BAC | 8 | 109-110/20 | 76 |
| 3 | p-Chlor-BTC | p-Chlor-BAC | 8 | 120-122/25 | 84 |
| 4 | o,p-Dichlor-BTC | o,p-Dichlor-BAC | 8 | 132-135/20 | 78 |
| 5 | m-Fluor-BTC | m-Fluor-BAC | 10 | 94/30 | 72 |
| 6 | p-Fluor-BTC | p-Fluor-BAC | 10 | 81- 83/20 | 78 |
| 7 | o-Fluor-o'-Chlor-BTC | o-Fluor-o'-Chlor-BAC | 10 | 95/16 | 78 |
| 8 | o,o'-Difluor-BTC | o,o'-Difluor-BAC | 10 | 87/20 | 83 |
| 9 | m-Methyl-BTC | m-Methyl-BAC | 8 | 115-117/30 | 58 |
| 10 | o-Trifluormethyl-BTC | o-Trifluormethyl-BAC | 10 | 100-102/40 | 75 |
| 11 | m-Trifluormethyl-BTC | m-Trifluormethyl-BAC | 10 | 98- 99/40 | 95 |
| 12 | p-Trifluormethyl-BTC | p-Trifluormethyl-BAC | 10 | 98-100/30 | 89 |
| 13 | m-Oxitrifluormethyl-BTC | m-Oxitrifluormethyl-BAC | 5 | 103-105/20 | 52 |
| 14 | o-Chlor-m-Trifluormethyl-BTC | o-Chlor-m-Trifluormethyl-BAC | 6 | 106-108/20 | 68 |
| 15 | m-Nitro-m'-Trifluormethyl-p-chlor-BTC | m-Nitro-m'-Trifluormethyl-p-chlor-BAC | 7 | 102-104/ 0,2 | 72 |
| 16 | o-Cyano-BTC | o-Cyano-BAC | 8 | 155-156/20 (Fp.: 37-38) | 95 |
| 17 | m-Cyano-BTC | m-Cyano-BAC | 8 | 158-160/18 | 81 |
| 18 | p-Cyano-BTC | p-Cyano-BAC | 8 | 139-140/18 | 78 |

*Beispiel 19* (Reduktion mit Butanthiol)

19,5 g (0,1 Mol) Benzotrichlorid, 18 g (0,2 Mol) Butanthiol und 0,2 g Kupfer-(I)-bromid wurden unter Rühren bis zum Rückfluss aufgeheizt und bis zum Ende der Chlorwasserstoff-Entwicklung (Dauer 6 h) reagieren gelassen. Anschliessend wurde fraktioniert destilliert und man erhielt 11,9 g (74%) Benzalchlorid bei Kp. 102-106°C/30 mbar.

*Beispiel 20* (Reduktion mit Thiophenol und CuBr in Toluol)

19,5 g (0,1 Mol) Benzotrichlorid, 22 g (0,2 Mol) Thiophenol und 0,2 g Kupfer-(I)-bromid in 40 ml Toluol wurden langsam aufgeheizt und bis zum Ende der Gasentwicklung (Dauer 10 h) bei 120°C (Ölbadtemperatur) reagieren gelassen. Nach dem Abdestillieren des Lösungsmittels wurden aus dem Reaktionsgemisch 12,7 g (79%) Benzalchlorid bei Kp. 100-104°C/30 mbar abdestilliert.

**Patentansprüche**

1. Verfahren zur Herstellung von Benzalchloriden aus Benzotrichloriden, dadurch gekennzeichnet, dass man Benzotrichloride mit Thiolen in Gegenwart von Kupfer-(I)-bromid bei erhöhten Temperaturen umsetzt.

2. Verfahren nach Anspruch 1, dadurch gekennzeichnet, dass man ein Benzotrichlorid der Formel

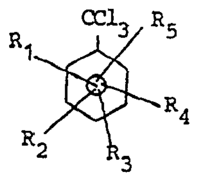

einsetzt, in der

$R_1$ bis $R_5$ unabhängig voneinander für Wasserstoff, Nitro, Halogen, Alkyl, Fluoralkyl, Aryl, substituiertes Aryl, O-Alkyl, O-Fluoralkyl, O-Aryl, O-substituiertes Aryl und/oder Cyanid stehen.

3. Verfahren nach Ansprüchen 1 und 2, dadurch gekennzeichnet, dass man ein Benzotrichlorid der Formel

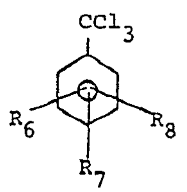

einsetzt, in der

$R_6$ bis $R_8$ unabhängig voneinander für Wasserstoff, Nitro, Fluor, Chlor, Cyanid, $C_1$-$C_4$-Alkyl, $C_1$-$C_4$-Fluoralkyl und/oder O-$C_1$-$C_4$-Fluoralkyl stehen.

4. Verfahren nach Ansprüchen 1 bis 3, dadurch gekennzeichnet, dass man pro Mol eines Benzotrichlorides 2 Mol eines Thiols einsetzt.

5. Verfahren nach Ansprüchen 1 bis 4, dadurch gekennzeichnet, dass man aliphatische Thiole mit 4 bis 10 C-Atomen oder aromatische Thiole mit 6 bis 8 C-Atomen einsetzt.

6. Verfahren nach Ansprüchen 1 bis 5, dadurch gekennzeichnet, dass man Thiophenol einsetzt.

7. Verfahren nach Ansprüchen 1 bis 6, dadurch gekennzeichnet, dass man in Gegenwart von 0,01 bis 10 Gew.-% Kupfer-(I)-bromid, bezogen auf das eingesetzte Benzotrichlorid, arbeitet.

8. Verfahren nach Ansprüchen 1 bis 7, dadurch gekennzeichnet, dass die Reaktionstemperatur zwischen der Temperatur des Beginns der Chlorwasserstoffentwicklung und der Temperatur liegt, bei der das Reaktionsgemisch unter Rückfluss siedet.

**Claims**

1. Process for the preparation of benzal chlorides from benzotrichlorides, characterised in that benzotrichlorides are reacted with thiols in the presence of copper(I) bromide at elevated temperatures.

2. Process according to Claim 1, characterised in that a benzotrichloride of the formula

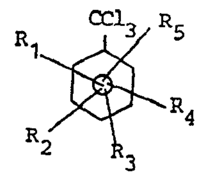

is used in which

$R_1$ to $R_5$ independently of one another represent hydrogen, nitro, halogen, alkyl, fluoroalkyl, aryl, substituted aryl, O-alkyl, O-fluoroalkyl, O-aryl, O-substituted aryl and/or cyanide.

3. Process according to Claims 1 and 2, characterised in that a benzotrichloride of the formula

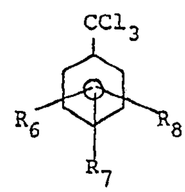

is used in which

$R_6$ to $R_8$ independently of one another represent hydrogen, nitro, fluorine, chlorine, cyanide, $C_1$-$C_4$-alkyl, $C_1$-$C_4$-fluoroalkyl and/or O-$C_1$-$C_4$-fluoroalkyl.

4. Process according to Claims 1 to 3, characterised in that 2 moles of a thiol are used per mole of a benzotrichloride.

5. Process according to Claims 1 to 4, characterised in that aliphatic thiols with 4 to 10 C atoms or aromatic thiols with 6 to 8 C atoms are used.

6. Process according to Claims 1 to 5, characterised in that thiophenol is used.

7. Process according to Claims 1 to 6, characterised in that the reaction is carried out in the presence of 0.01 to 10% by weight of copper(I) bromide, based on the benzotrichloride used.

8. Process according to Claims 1 to 7, characterised in that the reaction temperature is between the temperature of the start of the evolution of hydrogen chloride and the temperature at which the reaction mixture boils under reflux.

## Revendications

1. Procédé de production de chlorures de benzylidène à partir de chlorures de benzényle, caractérisé en ce qu'on fait réagir des chlorures de benzényle avec des thiols en présence de bromure de cuivre (I) à des températures élevées.

2. Procédé suivant la revendication 1, caractérisé en ce qu'on utilise un chlorure de benzényle de formule

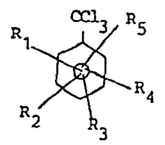

dans laquelle

$R_1$ à $R_5$ représentent indépendamment les uns des autres de l'hydrogène, un groupe nitro, un halogène, un groupe alkyle, fluoralkyle, aryle, aryle substitué, O-alkyle, O-fluoralkyle, O-aryle, O-aryle substitué et/ou cyanure.

3. Procédé suivant les revendications 1 et 2, caractérisé en ce qu'on utilise un chlorure de benzényle de formule

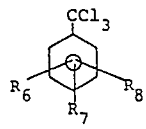

dans laquelle

$R_6$ à $R_8$ représentent indépendamment les uns des autres l'hydrogène, un groupe nitro, du fluor, du chlore, un groupe cyanure, alkyle en $C_1$ à $C_4$, fluoralkyle en $C_1$ à $C_4$ et/ou O-fluoralkyle en $C_1$ à $C_4$.

4. Procédé suivant les revendications 1 à 3, caractérisé en ce qu'on utilise 2 mol d'un thiol par mole d'un chlorure de benzényle.

5. Procédé suivant les revendications 1 à 4, caractérisé en ce qu'on utilise des thiols aliphatiques ayant 4 à 10 atomes de carbone ou des thiols aromatiques ayant 6 à 8 atomes de carbone.

6. Procédé suivant les revendications 1 à 5, caractérisé en ce qu'on utilise le thiophénol.

7. Procédé suivant les revendications 1 à 6, caractérisé en ce qu'on opère en présence de 0,01 à 10% en poids de bromure de cuivre (I) par rapport au chlorure de benzényle utilisé.

8. Procédé suivant les revendications 1 à 7, caractérisé en ce que la température de réaction se situe entre la température du début de dégagement du chlorure d'hydrogène et la température à laquelle le mélange réactionnel bout au reflux.